Europäisches Patentamt

European Patent Office

Office européen des brevets

(19)

(11) **EP 0 942 281 A2**

(12) **EUROPÄISCHE PATENTANMELDUNG**

(43) Veröffentlichungstag:
**15.09.1999 Patentblatt 1999/37**

(51) Int. Cl.$^6$: **G01N 33/00**, G01N 27/12

(21) Anmeldenummer: **99103014.9**

(22) Anmeldetag: **15.02.1999**

(84) Benannte Vertragsstaaten:
**AT BE CH CY DE DK ES FI FR GB GR IE IT LI LU
MC NL PT SE**
Benannte Erstreckungsstaaten:
**AL LT LV MK RO SI**

(30) Priorität: **13.03.1998 DE 19811069**

(71) Anmelder:
**SIEMENS AKTIENGESELLSCHAFT
80333 München (DE)**

(72) Erfinder:
• **Bausewein, Andreas
85416 Langenbach (DE)**
• **Meixner, Hans
85540 Haar (DE)**
• **Pohle, Roland
81739 München (DE)**
• **Chemisky, Eric
85540 Haar (DE)**
• **Scherg, Thomas
85591 Vaterstetten (DE)**

(54) **Messschaltung zur Ermittlung der Konzentration eines oder mehrerer Gase in einem Gasgemisch**

(57)   Eine Meßschaltung (25) weist ein Gas-Sensorelement (32) mit variablem Widerstand auf, dessen Widerstand sich in Abhängigkeit der Konzentration eines Gases in dem Gasgemisch verändert. Eine Meßsignalerzeugungsstufe (2; 26), eine Meßsignalumwandlungsstufe (3; 27) und eine Meßstufe (28) der Meßschaltung (25) sind so zusammengeschaltet, daß diese einen Schwingkreis bilden, dessen charakteristische Schwingungseigenschaften sich mit der Veränderung des Widerstands des Gas-Sensorelements (32) ändert.

**FIG 3**

EP 0 942 281 A2

**Beschreibung**

Die Erfindung betrifft eine Meßschaltung zur Ermittlung der Konzentration eines oder mehrerer Gase in einem Gasgemisch.

Gattungsgemäße Meßschaltungen weisen einen Schwingkreis auf, der ein Gas-Sensorelement mit variablem Widerstand umfaßt, wobei sich der Widerstand des Gas-Sensorelements und damit die Eigenfrequenz des Schwingkreises in Abhängigkeit der Konzentration des Gases bzw. der Gase in dem Gasgemisch verändert. Die WO 95/29072 offenbart eine Meßschaltung, bei der ein zu beheizendes Gas-Sensorelement zusammen mit einem als Oszillator beschalteten Timerbaustein verwendet wird. In dieser Beschaltung wird das Gas-Sensorelement mit einem scharfen Rechtecksignal beaufschlagt, wobei der Timerbaustein so beschaltet ist, daß sich die Frequenz des Rechtecksignals, mit der das Gas-Sensorelement beaufschlagt wird, in Abhängigkeit von dessen Widerstand verändert. Die Frequenz des sich so ergebenden Rechtecksignals kann gemessen werden, wobei sich daraus ein Rückschluß auf den momentanen Widerstand des Gas-Sensorelements ergibt. Aus dem momentanen Widerstand des Gas-Sensorelements kann auf den Gehalt eines oder mehrerer Gase eines Gasgemischs geschlossen werden, das auf das Gas-Sensorelement einwirkt.

Weiterhin sind Meßschaltungen bekannt, die zwei unterschiedliche Gas-Sensorelemente aufweisen, von denen ein Gas-Sensorelement zur Erfassung oxidierbarer Gase geeignet ist, während das andere Gas-Sensorelement zur Erfassung reduzierbarer Gase dient. Mit solchen Meßschaltungen können sogenannte "Maskierungseffekte" eliminiert werden, die bei der Verwendung eines einzigen Sensorelements auftreten.

Bei allen vorbekannten Meßschaltungen ist von Nachteil, daß nur ungenaue Messungen über die Konzentration eines oder mehrerer Gase in einem Gasgemisch möglich sind.

Es ist daher Aufgabe der Erfindung, eine Meßschaltung zur Ermittlung der Konzentration eines oder mehrerer Gase in einem Gasgemisch bereitzustellen, die schnell, genau und zuverlässig arbeitet.

Diese Aufgabe wird gemäß der Erfindung dadurch gelöst, daß der Signalgenerator die Gas-Sensorelemente mit einem Meßsignal beaufschlagt, dessen Ableitung nach der Zeit im wesentlichen stetig verläuft. Dabei weist ein Signal, dessen Ableitung nach der Zeit im wesentlichen stetig verläuft, keine oder nur sehr geringe "Knicke" in seinem Verlauf auf, die scharfe Kanten, einfache kanten oder sprunghafte Änderungen sein können. "Knicke" sind insbesondere auch deutliche Spitzen des Meßsignals, die auch als "Peaks" bezeichnet werden. Im Bereich um solche Peaks herum hat die Ableitung des Signals nach der Zeit auf einer Seite des Peaks einen sehr großen positiven Wert, während die Ableitung auf der anderen Seite des Peaks einen sehr kleinen, insbesondere negativen Wert annimmt. Aber auch andere Knicke im Meßsignal bewirken, daß die Ableitung des Meßsignals nach der Zeit eine Unstetigkeit aufweisen, da sich in solchen Knicken die Steigung des Meßsignals abrupt ändert.

Dieser Aspekt der Erfindung beruht auf der Erkenntnis, daß Halbleiter-Gas-Sensorelemente sehr empfindlich auf abrupte Änderungen der Steigung des Meßsignals reagieren. Gemäß der Erfindung ist daher vorgesehen, daß das Meßsignal eine Form annimmt, die durch einfache Eingriffe und/oder Änderungen in der Meßschaltung definierbar ist. Signale mit Sinusform, mit der Form eines Rechtecksignals mit abgerundeten Flanken oder mit einer Trapezform haben sich als geeignet erwiesen.

Weiterhin ist es von Vorteil, wenn das Meßsignal über den gesamten Meßbereich des Gas-Sensorelements eine konstante Form aufweist, weil sich so ein besonders genauer Betrieb der Gas-Sensorelemente ergibt. Damit ist gemeint, daß die graphisch dargestellten Verläufe der Meßsignale für unterschiedliche Meßwerte der Gas-Sensorelemente jeweils zueinander ähnlich sind.

Zusätzlich zu wenigstens einer der vorstehenden Ausbildungen oder alternativ dazu hat es sich als besonders vorteilhaft herausgestellt, wenn das Meßsignal mit einer Amplitude erzeugt wird, die im wesentlichen über den gesamten genutzten Meßbereich des Gas-Sensorelements bzw. der Gas-Sensorelemente konstant ist. Die Erfindung beruht nämlich auch auf dem Grundgedanken, daß durch die Beaufschlagung des Gas-Sensorelements mit einer konstanten Meßspannungsamplitude und/oder konstanten Form über den gesamten Meßbereich des Gas-Sensorelements eine besonders genaue Abtastung der Konzentration eines Gases in einem Gasgemisch erfolgen kann.

Die erfindungsgemäße Meßschaltung arbeitet besonders zuverlässig mit resistiven Metalloxid-Gas-Sensorelementen. Resistive Metalloxid-Gas-Sensorelemente zeigen bei hohen Betriebstemperaturen eine deutliche Abhängigkeit des Schichtwiderstands von der umgebenden Gasatmosphäre. Es hat sich herausgestellt, daß bei der Messung des Widerstands eines Gas-Sensorelements besonders zuverlässige Ergebnisse erhalten werden, wenn der zu messende Sensorwiderstand als Teil einer Oszillatorschaltung ausgeführt ist, bei der der sich verändernde Widerstand des Gas-Sensorelements in eine Frequenzänderung umgesetzt wird. Im Gegensatz zur Messung des Widerstandes des Gas-Sensorelements mit einem konstanten DC-Meßstrom oder mit einer konstanten DC-Meßspannung werden hierbei zuverlässige und gute Ergebnisse erzielt, wobei der Einfluß von Störgrößen verringert wird. Dies wird darauf zurückgeführt, daß bei den erfindungsgemäßen Ausbildungen der Meßschaltungen der Effektivwert des elektrischen Feldes zwischen den Meßelektroden des Gas-Sensorelements unabhängig von der Zusammensetzung des zu messenden Gasgemisches im wesentlichen konstant bleibt, so daß sogenannte "Polarisationseffekte" verhindert werden. Zu diesem Zweck wird gemäß der Erfindung ein alternierendes Meßsignal verwendet, das keinen oder nur einen geringen Gleichspannungsanteil aufweist. Durch die erfindungsgemäße Ausbildung der Meßschaltung als Schwingkreis wird diese Bedingung immer erfüllt, wenn das Gas-Sensorelement beispielsweise zum Laden und Entladen eines Kondensators eingesetzt wird. In diesem Fall kompensieren sich die Lade- und Entladeströme immer, da sich ansonsten der im Schwingkreis vorgesehene Kondensator mit der Zeit aufladen würde und Oszillationen des Schwingkreises zum Erliegen kämen.

Wenn die Amplitude und/oder die Form des Meßsignals über den Meßbereich des Gas-Sensorelements im wesentlichen konstant ist, dann kann die Meßspannung klein gehalten werden. Gerade für resistive Metalloxid-Gas-Sensoren hat sich eine Amplitude der Meßspannung im Voltbereich und typischerweise von 1 Volt als vorteilhaft herausgestellt. Die Schaltung eignet sich besonders für die Widerstandsmessung auch von hohen Widerständen mit sehr kleinen Spannungen. Die starke Begrenzung der Meßspannung hängt mit dem Abstand zwischen den Sensorelektroden zusammmen, der typischerweise 20 µm beträgt sowie von der Tatsache, daß zu hohe Feldstärken die Eigenschaften der Sensoren beeinträchtigen. Den Schwierigkeiten der Messung mit einer so kleinen Meßspannung kann durch die erfindungsgemäße Ausgestaltung auf einfache Weise begegnet werden, da mit dieser Ausgestaltung sogar Sensorwiderstände zwischen 100 kΩ und 5 MΩ gemessen werden können, bei denen die zu messenden resultierenden Ströme im µA-Bereich und darunter liegen.

Dabei ist besonders von Bedeutung, daß durch die Ausgestaltung der erfindungsgemäßen Meßschaltung mit einem Meßsignal, das keine oder nur gering ausgeprägte Knicke und Flanken aufweist, wesentliche Ionenbewegungen in der Sensorschicht verhindert werden, die zu Polarisationseffekten und zu einem Langzeitdrift des Sensorsignals führen können. Gerade beim Einsatz von resistiven Metalloxid-Gas-Sensoren, die einerseits einen hohen Sensorwiderstand im Bereich von einigen 100kΩ haben und die andererseits bei hohen Betriebstemperaturen von ca. 600°C arbeiten, ist dies von Vorteil.

Gemäß einem weiteren Aspekt der Erfindung weist die Meßschaltung die folgenden Stufen auf:

- eine Meßsignalerzeugungsstufe zur Erzeugung eines alternierenden Grundsignals,
- eine Meßsignalumwandlungsstufe zur Umwandlung des Grundsignals in ein Meßsignal und
- eine Meßstufe mit dem Gas-Sensorelement,

wobei ferner die Meßsignalerzeugungsstufe und/oder die Meßsignalumwandlungsstufe und die Meßstufe so zusammengeschaltet sind, daß diese einen einzigen Schwingkreis bilden, dessen charakteristische Schwingungseigenschaften und insbesondere dessen Grundfrequenz sich mit der Veränderung des Widerstands des Gas-Sensorelements verändert. Bei dieser Ausbildung der erfindungsgemäßen Meßschaltung lassen sich die Veränderungen des Widerstands des Gas-Sensorelements auf besonders einfache und zuverlässige Weise anhand einer Änderung der Schwingungsfrequenz des Schwingkreises abtasten.

Erfindungsgemäß weist die Meßsignalerzeugungsstufe einen als Fensterkomparator mit Hysterese ausgebildeten Operationsverstärker auf. Dies hat sich als besonders vorteilhaft herausgestellt, weil sich mit einem solchen Operationsverstärker auf einfache Weise saubere Oszillationen in einem Schwingkreis auslösen lassen. Darüber hinaus wird mit einer solchen Meßsignalerzeugungsstufe ein Grundsignal erzeugt, dessen Amplitude und Grundform in weitem Umfang unabhängig von der Belastung konstant bleibt.

Wenn zusätzlich oder alternativ zu der vorstehenden Ausgestaltung der Meßsignalerzeugungsstufe die Meßsignalumwandlungsstufe als Bandpassfilterstufe oder als Tiefpaßstufe ausgebildet ist, dann kann auf einfache Weise gewährleistet werden, daß das aus dem Grundsignal umgewandelte Meßsignal keine wesentlichen Knicke aufweist. Damit können selbst steile Flanken eines von der Meßsignalerzeugungsstufe erzeugten Rechtecksignals so abgemildert werden, daß das Gas-Sensorelement von etwaigen Polarisationseffekten verschont bleibt. Durch die Verringerung der Signal-Bandbreite werden nämlich Oberwellen ausgelöscht, so daß sich flache Flanken des Meßsignals ergeben.

Wenn die Meßstufe einen als Integrator geschalteten Operationsverstärker aufweist, dessen erster Eingang mit dem Gas-Sensorelement verbunden ist und dessen zweiter Eingang mit dem Ausgang der Meßsignalumwandlungsstufe verbunden ist, dann ergibt sich gerade in Verbindung mit einem hochohmigen Halbleiter-Gas-Sensorelement eine besonders zuverlässige Auswertung der Konzentration eines Gases in einem Gasgemisch. In einem solchen Fall wird nämlich das Gas-Sensorelement vorzugsweise zwischen der Systemmasse und dem vorgesehenen Eingang des Operationsverstärkers angeordnet. Bei hohen Betriebstemperaturen werden nicht zu vernachlässigende Einstreuungen von Störsignalen durch eine Systemheizung zur Systemmasse abgeleitet, so daß sie nicht durch das Sensorsubstrat fließen. Der Operationsverstärker regelt diesen Eingang nach, und zwar auf das Niveau des anderen Eingangs, an dem das Meßsignal anliegt. Aufgrund dieser Anordnung des Gas-Sensorelements zwischen der "elektrisch kalten" Systemmasse, die aufgrund ihrer niedrigen Impedanz etwaige Leckströme ableitet, und dem Eingang des entsprechenden Operationsverstärkers ergibt sich eine besonders genaue Messung insbesondere der Konzentration von Stickoxiden. Durch die Anordnung des hochohmigen Sensors im Bereich der Systemmasse wird nämlich das Rauschen im Bereich des Halbleiter-Gas-Sensorelements vermindert.

Bei der Ausbildung der Meßstufe mit einem Operationsverstärker hat es sich weiterhin als vorteilhaft erwiesen, in einem Rückführzweig zwischen dem Ausgang und einem Eingang des Operationsverstärkers eine Kapazität vorzusehen. Mit dieser Kapazität kann auch eine Grundfrequenz der erfindungsgemäßen Meßschaltung eingestellt werden.

Abweichend von der vorstehend dargelegten Ausgestaltung der Meßstufe kann die Meßstufe auch einen als Verstärker geschalteten Operationsverstärker aufweisen, dessen erster Eingang mit dem Ausgang der Meßsignalumwandlungsstufe bzw. der Meßsignalerzeugungsstufe verbunden ist, wobei in einem Rückführzweig zwischen dem Ausgang und einem Eingang des Operationsverstärkers das zweite Gas-Sensorelement vorgesehen ist. Mit einer solchen Meßstufe können Änderungen des Widerstandes von eher niederohmigen Halbleiter-Gas-Sensorelementen zuverlässig ausgemessen werden. Solche niederohmigen Halbleiter-Gas-Sensorelemente werden insbesondere zur Messung der Konzentration von Kohlenwasserstoffen oder von Ammoniak verwendet.

Die Erfindung sieht weiterhin eine Meßschaltung vor, die ein erstes und ein zweites Gas-Sensorelement mit variablem Widerstand aufweist, wobei eine Meßsignalerzeugungsstufe zur Erzeugung eines alternierenden Grundsignals, eine Meßsignalumwandlungsstufe zur Umwandlung des Grundsignals in ein Meßsignal sowie eine erste Meßstufe mit dem ersten Gas-Sensorelement und eine zweite Meßstufe mit dem zweiten Gas-Sensorelement vorgesehen sind. Die Meßsignalerzeugungsstufe und/oder die Meßsignalumwandlungsstufe sowie die erste Meßstufe und die zweite Meßstufe sind dabei so zusammengeschaltet, daß sie einen einzigen Schwingkreis bilden, dessen charakteristische Schwingungseigenschaften und insbesondere dessen Grundfrequenz sich mit der Veränderung des Widerstands des ersten Gas-Sensorelements und/oder des zweiten Gas-Sensorelements ändern.

Mit einer solchen Meßschaltung mit zwei Gas-Sensorelementen können vorteilhafterweise Maskierungseffekte eines Gas-Sensorelements von normalen Meßeffekten unterschieden werden.

Solche Maskierungseffekte treten zum Beispiel auf, wenn in Abgasen eines Verbrennungsmotors neben oxidierbaren Gasen in hohem Ausmaß reduzierbare Gase vorhanden sind. Die in solchen Abgasen enthaltenen reduzierbaren Gase oxidieren die durch die oxidierbaren Gase anreduzierte gasempfindliche Schicht eines Halbleiter-Gas-Sensorelements wieder auf, so daß sich die reduzierende und die oxidierende Wirkung der in den Kraftfahrzeuggasen enthaltenen Gasanteile bei der Beaufschlagung des Gas-Sensorelements in starkem Ausmaß gegenseitig aufheben. Dies führt dazu, daß Fehlmessungen auftreten. Bei der erfindungsgemäßen Ausgestaltung mit zwei in einem einzigen Schwingkreis angeordneten Gas-Sensorelementen ergibt sich eine besonders einfache Trennung der jeweiligen Effekte. Dabei dient ein Gas-Sensorelement zur Erfassung oxidierbarer und damit reduzierender Gase, während das andere Gas-Sensorelement zum Erfassen reduzierbarer Gase dient.

Vorzugsweise wird ein Gas-Sensorelement zusammen mit einem als Integrator geschalteten Operationsverstärker verwendet, dessen erster Eingang mit dem Gas-Sensorelement verbunden ist, und dessen zweiter Eingang mit dem Ausgang der Meßsignalumwandlungsstufe verbunden ist. Diese Beschaltung dient für den Einsatz von hochohmigen Halbleiter-Gas-Sensorelementen, die insbesondere zur Messung der Konzentration von Stickoxiden bzw. von Kohlenmonoxid eingesetzt werden.

Niederohmige Gas-Sensorelemente wie beispielsweise für Kohlenwasserstoffe oder für Ammoniak werden dann im Zusammenhang mit einem als Verstärker geschalteten Operationsverstärker verwendet, dessen erster Eingang mit dem Ausgang des Operationsverstärkers des hochohmigen Gas-Sensorelements verbunden ist, während der zweite Eingang mit der Meßsignalumwandlungsstufe bzw. mit der Meßsignalerzeugungsstufe verbunden ist. Das niederohmige Gas-Sensorelement selbst ist vorzugsweise in einem Rückführzweig zwischen dem Ausgang und einem Eingang des Operationsverstärkers vorgesehen.

Bei der vorstehenden Ausgestaltung der Meßschaltung ergeben sich mehrere Vorteile. Zum einen laufen die Meßspannungen an den beiden Gas-Sensorelementen zueinander synchron, so daß Abschirmungen der beiden Gas-Sensorelemente gegeneinander grundsätzlich nicht notwendig sind. Abschirmungen können trotzdem vorgesehen werden, weil die Meßsignale von niederohmigen Gas-Sensorelementen die Meßsignale von hochohmigen Gas-Sensorelementen verfälschen können. Darüber hinaus hängt die Grundfrequenz der Meßschaltung bei der vorstehend dargelegten Beschaltung von dem Verhältnis der Widerstände der beiden Gas-Sensorelemente ab, so daß ein Ausgangssignal zur Anzeige von zwei Meßwerten ausreicht.

Die Amplitude und Form der Meßspannung ist gerade an dem hochohmigen Gas-Sensorelement über den Meßbereich des Gas-Sensorelements konstant, so daß sich ein polarisationsfreier Betrieb des hochohmigen Gas-Sensorelements ergibt.

In der vorstehenden Beschaltung braucht die Amplitude der Meßspannung an dem niederohmigen Gas-Sensorelement nicht unbedingt konstant sein, weil derartige Gas-Sensorelemente nicht übermäßig polarisationsempfindlich sind. Die Meßspannung am zweiten Sensor hat dieselbe Form wie die Meßspannung am ersten Sensor. Daher hat sie auch einen Mittelwert gleich Null.

In Weiterbildung der Erfindung ist zwischen der wie vorstehend dargelegten ersten Meßstufe mit dem ersten Gas-Sensorelement und der zweiten Meßstufe mit dem zweiten Gas-Sensorelement eine Potentialanpassungsstufe vorgesehen, die so ausgebildet ist, daß ein Anteil des Meßsignals am Ausgang der ersten Meßstufe unterdrückbar ist. Dadurch wird die Empfindlichkeit der erfindungsgemäßen Meßschaltung verbessert.

Die Potentialanpassungsstufe weist dabei vorteilhafterweise einen als Subtrahierer geschalteten zweiten Operationsverstärker auf, dessen erster Eingang mit dem Ausgang der ersten Meßstufe verbunden ist und dessen zweiter Eingang mit dem Ausgang der Meßsignalumwandlungsstufe verbunden ist, wobei in einem Rückführzweig zwischen dem Ausgang und einem Eingang des zweiten Operationsverstärkers ein Widerstand vorgesehen ist.

Die Erfindung ist auch in der Kombination einzelner Merkmale der jeweiligen unterschiedlichen unabhängigen Anspruchssätze verwirklicht.

Die erfindungsgemäße Meßschaltung ermöglicht auch die Verwendung von zwei Sensoren zur Auswertung der Luftgüte, die bei einer Konzentrationserhöhung des jeweils gemessenen Gases unterschiedliche Verhaltensrichtungen aufweisen. So kann ein Gas-Sensorelement verwendet werden, das bei dem Vorhandensein von $NO/NO_2$-Gasen eine Widerstanderhöhung zeigt, während das andere Gas-Sensorelement bei dem Vorhandensein von ungesättigten Kohlenwasserstoffen "Hc" eine Widerstanderniedrigung aufweist. Vorteilhafterweise wird die Meßschaltung so ausgebildet, daß die Ausgangsfrequenz linear vom Verhältnis der beiden Widerstände der Gas-Sensorelemente abhängt.

Dabei ist bei der erfindungsgemäßen Ausbildung von Vorteil, daß auch Sensoren eingesetzt werden können, die sehr empfindlich auf Polarisationseffekte reagieren. Die Meßspannung kann nämlich durch die erfindungsgemäße Beschaltung hinsichtlich ihrer Amplitude und ihrer Kurvenform genau festgelegt werden, so daß die Polarisation des entsprechenden Gas-Sensorelements minimierbar ist. Darüber hinaus ist von Vorteil, daß auch sehr hochohmige Sensoren verwendet werden können, da die Erfindung vorsieht, daß einer der Anschlüsse des Gas-Sensorelements mit der Systemmasse verbindbar ist, also mit einem "elektrisch kalten" Bereich der Meßschaltung.

Beachtung verdient die Ausgestaltung gemäß der Erfindung, bei der ein Anschluß eines Gas-Sensorelements mit einer insbesondere virtuellen Masse des Systems verbunden ist, weil dadurch die Stabilität der Meßschaltung deutlich erhöht wird. Der betreffende Anschluß des Gas-Sensorelements kann dann nämlich auch die Rolle einer sogenannten "Guard"-Leitung zwischen einer Sensorschicht und einem etwaigen Heizelement des Gas-Sensorelements übernehmen. Der Anschluß nur einer Elektrode eines Gas-Sensorelements kann auch bei der Messung mit Sensorarrays von Vorteil sein, da alle zu messenden Sensoren eine gemeinsame Elektrode besitzen können. Dadurch verringert sich die Anzahl von Anschlüssen, wobei weiterhin eine effektive elektrische Abschirmung des betreffenden Sensorarrays erreicht wird.

Außerdem gestattet es die erfindungsgemäße Meßschaltung, ein Gas-Sensorelement einzusetzen, das relativ polarisationsunempfindlich ist, wobei gerade bei dem Zusammenschalten von polarisationsempfindlichen und polarisationsunempfindlichen Gas-Sensorelementen eine besonders vorteilhafte, weil einfach auszuwertende Meßschaltung entsteht.

Es hat sich weiterhin als vorteilhaft herausgestellt, die Mittelpotentiale der Sensorschichten der Gas-Sensorelemente konstant zu halten, wobei es besonders vorteilhaft ist, sie auf 0 V zu halten, denn dies erlaubt den Einsatz der Meßschaltung auch bei höheren Temperaturen, beispielsweise wenn die Einstreuungen von Heizungen für die Gas-Sensorelemente in deren Sensorschichten nicht mehr zu vernachlässigen sind.

Weiterhin ist es von Vorteil, daß die Meßspannungen für die beiden Gas-Sensorelemente zueinander synchron sind, so daß Synchronisationseffekte zwischen den Meßsignalen an den beiden Gas-Sensorelementen schon prinzipiell nicht von Bedeutung sind.

Die erfindungsgemäße Meßschaltung mit zwei "gegenläufigen" Sensoren liefert für die Messung der Luftgüte bzw. der Konzentrationen von Gasen in zu messender Luft ein einziges Signal, was deren Anwendung gerade für Klimaanlagen von Kraftfahrzeugen begünstigt.

Schließlich ist es von Vorteil, wenn die Gas-Sensorelemente bei gleicher Temperatur betrieben werden und wenn die Aktivierungsenergie der Sensorschichten der Gas-Sensorelemente in etwa gleich groß ist. In einem solchen Fall ist eine exponentielle Temperaturabhängigkeit der Grundfrequenz der Meßschaltung stark reduziert.

Weiterhin ist es gemäß der Erfindung von Vorteil, wenn die Grundfrequenz der Meßschaltung viel höher liegt, als die Beweglichkeit der Ionen in den bekannten Halbleiter-Gas-Sensorelementen. Polarisationseffekten wird dadurch nämlich entgegengewirkt.

Gemäß der Erfindung kann die Form des Meßsignals einfach durch Abändern der Bauteile der Meßsignalumwandlungsstufe durchgeführt werden. Dadurch läßt sich die erfindungsgemäße Schaltung einfach auf Anforderungen im Betrieb anpassen.

Vorteilhafterweise ist die Grundfrequenz der Meßschaltung, also die Frequenz, mit der diese schwingt, wenn sich die Gas-Sensorelemente bzw. ein Gas-Sensorelement in reiner Luft befindet, leicht durch den Austausch eines Kondensators bzw. eines Widerstandes in einem weiten Bereich von 1 Hz bis 40000 Hz einstellbar. Diese Frequenz sollte aus Gründen der Meßgeschwindigkeit ausreichend hoch gewählt werden, weil bei gleichbleibender Auflösung die Meßzeit umgekehrt proportional zu der gewählten Grundfrequenz ist. Dies bedeutet, daß sich die Meßzeit verlängert, je kleiner die Meßfrequenz ist.

Die erfindungsgemäße Schaltung eignet sich demzufolge besonders für Gas-Sensorelemente, die bei ihrer optimalen Betriebstemperatur einen hohen gasempfindlichen Sensorwiderstand von typischerweise > 500 kΩ in synthetischer Luft haben und deren Substrat aufgrund der hohen verwendeten Betriebstemperatur von typischerweise > 600°C einen nicht zu vernachlässigenden Einfluß hat.

Die erfindungsgemäße Schaltung ist auch geeignet, wenn das Verhältnis zwischen zwei Sensorwiderständen berechnet werden soll. So können Gas-Sensorelemente verwendet werden, bei denen auf ein und demselben Halbleiter-Substrat zwei Gas-Sensorelemente vorgesehen sind. Das erste Gas-Sensorelement ist dann ein gewöhnliches resistives Gas-Sensorelement, dessen Widerstand von der Gaszusammensetzung und exponentiell von der Betriebstemperatur abhängt. Das zweite Gas-Sensorelement ist passiviert, was bedeutet, daß Gase nicht zu dessen Sensorschicht gelangen können. Dieser Sensor ist ebenfalls exponentiell temperaturempfindlich. Ein solches Gas-Sensorelement kann auch in der Weise aufgebaut sein, daß durch katalytische Aktivierung erreicht wird, daß es zwar prinzipiell auf verschiedene Gaszusammensetzungen reagiert, jedoch nur mit einem binären Signalsprung. Mit der Einschränkung, daß dieses Gas-Sensorelement nur Gaszusammensetzungen ausgesetzt ist, bei denen kein Signalsprung stattfindet, wie es beispielsweise bei einem Aussetzen des Gas-Sensorelements in mageres Abgas eines Verbrennungsmotors der Fall ist, verhält sich ein solches Gas-Sensorelement wie ein passiviertes Gas-Sensorelement. Unter der Voraussetzung, daß das Temperaturverhalten der Widerstände der beiden Gas-Sensorelemente dasselbe ist, und daß beide Gas-Sensorelemente dieselbe Aktivierungsenergie besitzen, kann durch die Berechnung des Verhältnisses zwischen zwei Gas-Sensorelement-Widerständen die Temperaturempfindlichkeit des Systems deutlich verringert werden.

Die Berechnung des Widerstandsverhältnisses kann mit zwei Exemplaren der vorgestellten Meßschaltung und einem einfachen Timer beispielsweise dem Zählerteil eines Mikrocontrollers auf einfache Weise realisiert werden. Das erste Sensorsignal, nämlich die Frequenz der ersten Meßschaltung, wird mit einem Zählereingang verbunden. Das andere Sensorsignal, nämlich die Frequenz der zweiten Meßschaltung, wird über einen Frequenzteiler mit dem Teilungsfaktor n mit einem "Gate"-Eingang des Zählers verbunden. Als digitales Ergebnis wird von dem Mikroprozessor der Zählerstand des Timers eingelesen. Dieser Zählerstand entspricht direkt dem Verhältnis der Frequenzen der ersten Meßschaltung und der zweiten Meßschaltung, multipliziert mit dem Teilungsfaktor n. Unter der Voraussetzung, daß die Schaltungen eine lineare Abhängigkeit zwischen der Sensorleitfähigkeit und der Ausgangsfrequenz aufweisen, ist das digitale Ergebnis proportional zum Widerstandverhältnis der Widerstände der beiden Gas-Sensorelemente in der ersten Meßschaltung und in der zweiten Meßschaltung und es kann als temperaturunempflindliches, jedoch gasempfindliches Signal behandelt werden.

Abweichend davon können auch zwei Meßschaltungen mit Gas-Sensorelementen verwendet werden, die auf unterschiedliche Gase in Gasgemischen reagieren. Damit kann dann auf einfache Weise ein Maskierungseffekt eliminiert werden, wie obenstehend in anderer Ausgestaltung analog beschrieben wurde. Dann muß natürlich sichergestellt werden, daR die beiden Oszillatorschaltungen gegeneinander abgeschirmt werden, damit sich die Meßschaltungen nicht ungewollt aufeinander synchronisieren, was zu unsinnigen Meßergebnissen führt.

Die Erfindung ist in der Zeichnung anhand mehrerer Ausführungsbeispiele veranschaulicht.

Figur 1    zeigt ein Blockschaltbild einer ersten erfindungsgemäßen Meßschaltung,

Figur 2    zeigt einen Schaltplan einer zweiten erfindungsgemäßen Meßschaltung,

Figur 3    zeigt ein Blockschaltbild einer dritten erfindungsgemäßen Meßschaltung,

Figur 4    zeigt ein Schaltbild einer vierten erfindungsgemäßen Meßschaltung und

Figur 5    zeigt ein Blockschaltbild einer weiteren erfindungsgemäßen Meßschaltung.

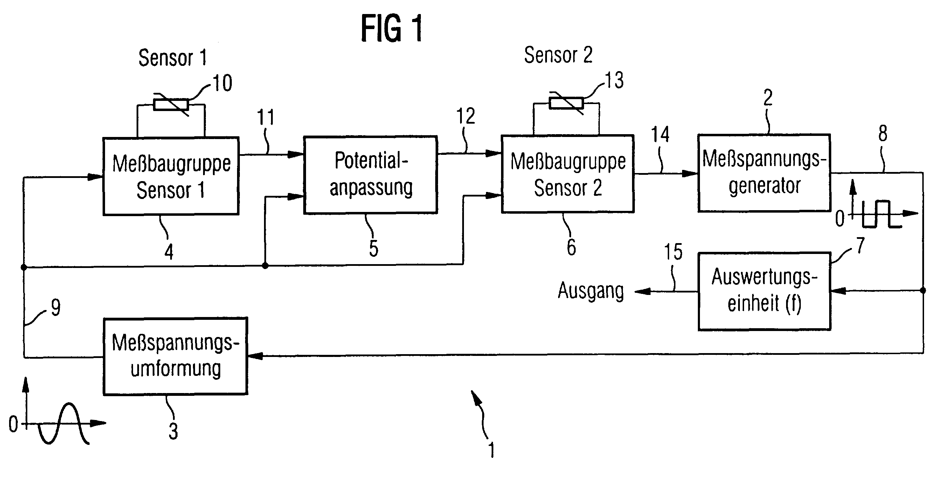

Figur 1 zeigt eine erste Meßschaltung 1 in der Ansicht als Blockschaltbild. Die Meßschaltung 1 stellt einen Schwingkreis dar, der aus einem Meßspannungsgenerator 2, einem Meßspannungsumformungsblock 3, einer ersten Meßbaugruppe 4, einer Potentialanpassungsstufe 5 und einer zweiten Meßbaugruppe 6 besteht. Im Bereich zwischen Meßspannungsgenerator 2 und Meßspannungsumformungsblock 3 ist eine Auswertungseinheit 7 vorgesehen.

Der Meßspannungsgenerator 2 erzeugt ein alternierendes Rechtecksignal, das symmetrisch um das Potential 0 herum schwingt und das nachfolgend als Grundsignal bezeichnet wird. Dieses Grundsignal wird auf eine Grundsignalleitung 8 ausgegeben und gelangt zum Meßspannungsumformungsblock 3. Dort werden die Flanken des Grundsignals so umgeformt, daß sich eine Sinusschwingung ergibt, die nachfolgend als Meßsignal bezeichnet wird. Dieses Meßsignal wird auf der Meßsignalleitung 9 ausgegeben. Das Meßsignal gelangt daraufhin zur ersten Meßbaugruppe 4, wo es von einem ersten Gas-Sensorelement 10 beaufschlagt wird. Darüber hinaus wird das Meßsignal der Potentialanpassungsstufe 5 und der zweiten Meßbaugruppe 6 zugeleitet.

Das Meßsignal wird von der ersten Meßbaugruppe 4 beeinflußt und von einem ersten Messungsanteil überlagert auf einer ersten Messungsausgangsleitung 11 an die Potentialanpassungsstufe 5 ausgegeben. In der Potentialanpassungsstufe 5 wird von diesem Signal das Meßsignal wieder subtrahiert, so daß auf einer Potentialanpassungsleitung 12 nur der mittelwertfreie Anteil der ersten Meßbaugruppe 4 ausgegeben wird.

In der zweiten Meßbaugruppe 6 beaufschlagt das zweite GasSensorelement 13 ebenfalls das Meßsignal von der Meßsignalleitung 9 und gibt es auf einer zweiten Ausgangsleitung 14 aus. Auf der zweiten Ausgangsleitung 14 wird auch der Anteil der Veränderung des Meßsignals durch die Zweite Meßbaugruppe 6 ausgegeben. Aufgrund der Rückwirkung dieser Informationen auf den Meßspannungsgenerator 2 stellt sich in der ersten Meßschaltung 1 eine Grundfrequenz ein, die von dem jeweiligen momentanen Widerstand des ersten Gas-Sensorelements 10 und des zweiten Gas-Sensorelements 13 abhängt. Diese Grundfrequenz wird von der Auswertungseinheit 7 erfaßt, die mit der Grundsignalleitung 8 in Verbindung steht. Die Auswertungseinheit 7 weist zur Ausgabe des Meßergebnisses einen Ausgang 15 auf, der von einer hier nicht gezeigten Vorrichtung wie beispielsweise einer Klimaanlage abgetastet werden kann.

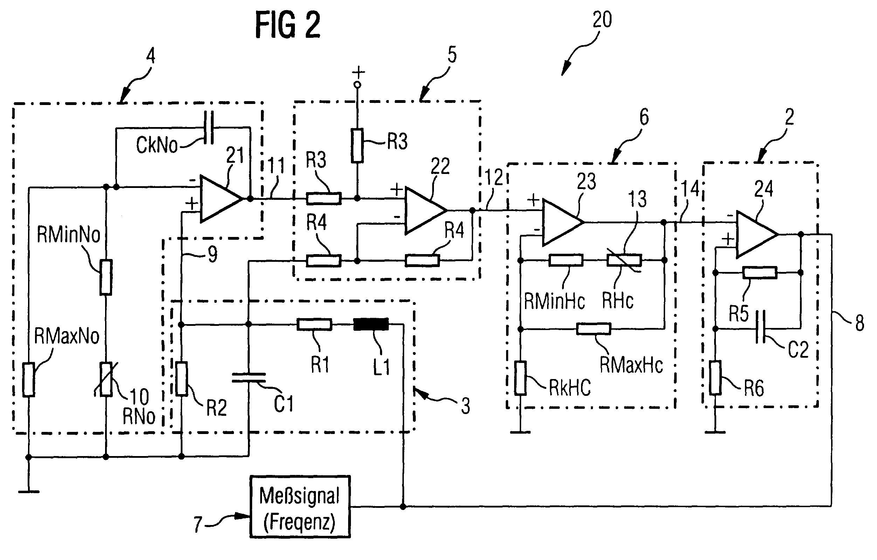

Figur 2 zeigt eine zweite Meßschaltung 20, die hinsichtlich ihres grundsätzlichen Aufbaus im wesentlichen mit der ersten Meßschaltung 1 aus Figur 1 übereinstimmt. Gleichen Baugruppen sind daher dieselben Bezugsziffern gegeben.

Im Einzelnen haben die Komponenten der zweiten Meßschaltung 20 den folgenden Aufbau.

Die erste Meßbaugruppe 4 hat einen ersten Operationsverstärker 21, der als Integrator mit einem in den Rückführzweig zwischen Ausgang und invertierendem Eingang geschalteten Kondensator CkNo versehen ist.

Mit der Kapazität CkNo am ersten Operationsverstärker 21 kann die Grundfrequenz der zweiten Meßschaltung 20 je nach verwendetem ersten Gas-Sensorelement 10 kalibriert werden.

Der erste Operationsverstärker 21 steht über die erste Ausgangsleitung 11 mit der Potentialanpassungsstufe 5 in Verbindung.

Zwischen den invertierenden Eingang des ersten Operationsverstärkers 21 und die Systemmasse ist das erste Gas-Sensorelement 10 mit dem Widerstand RNo geschaltet. Dabei ist das erste Gas-Sensorelement 10 in Reihe mit einem Widerstand RMinNo geschaltet, der den invertierenden Eingang des ersten Operationsverstärkers 21 mit einem Mindesteingangswiderstand beaufschlagt, wenn der Widerstand des ersten Gas-Sensorelements 10 sehr klein wird. Parallel zum ersten Gas-Sensorelement 10 und zum Widerstand RMinNo ist ein Widerstand RMaxNo geschaltet, der einen Maximalwert für einen Eingangswiderstand des invertierenden Eingangs des ersten Operationsverstärkers 21 bereitstellt, wenn der Widerstand des ersten Gas-Sensorelements 10 im Betrieb sehr groß werden sollte. Daraus ergibt sich eine Oszillationsgarantie der Meßschaltung 1 für einen großen Bereich von Umweltbedingungen.

Durch die Bereitstellung der Widerstände RMaxNo und RMinNo wird die minimale und maximale Frequenz der zweiten Meßschaltung 20 auch bei einer dramatischen Veränderung des ersten Gas-Sensorelements 10 infolge einer plötzlichen und starken Gasbeaufschlagung begrenzt. Die Verhältnisse der drei Widerstände RMinNo, RMaxNo und RNo sind so gewählt, daß sowohl bei einer extremen Niederohmigkeit als auch bei einer extremen Hochohmigkeit des Widerstands RNo des Gas-Sensorelements 10 der Gruppenwiderstand der Widerstände RMinNo, RMaxNo und RNo stets innerhalb eines einfach zu berechnenden Bereichs bleibt. Hierdurch ist sichergestellt, daß sich die Frequenz des Ausgangssignals nicht in einen Bereich verschieben kann, der außerhalb des Aufnahmebereichs einer nachgeschalteten Auswerteeinheit liegt.

Die Potentialanpassungsstufe 5 weist einen zweiten Operationsverstärker 22 auf, der als Subtrahierer mit einem Widerstand R4 zwischen seinem Ausgang und seinem invertierenden Eingang beschaltet ist. Der invertierende Eingang des zweiten Operationsverstärkers 22 ist über je einen Widerstand R3 mit der Versorgungsspannung bzw. mit der ersten Ausgangsleitung 11 verbunden. Der nicht-invertierende Eingang des zweiten Operationsverstärkers 22 ist über einen Widerstand R4 mit dem Meßspannungsumformungsblock 3 verbunden. Der Ausgang des zweiten Operationsverstärkers 22 führt über die Potentialanpassungsleitung 12 zur zweiten Meßbaugruppe 6.

Die zweite Meßbaugruppe 6 weist einen dritten Operationsverstärker 23 auf. Der nicht-invertierende Eingang des dritten Operationsverstärkers 23 ist über die Potentialanpassungsleitung 12 mit dem Ausgang des zweiten Operationsverstärkers 22 der Potentialanpassungsstufe 5 verbunden. In der Rückführleitung zwischen dem Ausgang des dritten Operationsverstärkers 23 und seinem invertierenden Eingang ist eine Anordnung aus dem zweiten Gas-Sensorelement 13, einem dazu in Reihe geschalteten Widerstand RMinHc sowie einem parallelen Widerstand RMaxHc vorgesehen. Der invertierende Eingang des dritten Operationsverstärkers 23 ist über einen Widerstand RkHc mit der Systemmasse verbunden.

Die Widerstände RMinHc und RMaxHc stellen sicher, daß sowohl bei einer extremen Niederohmigkeit als auch bei einer extremen Hochohmigkeit des zweiten Gas-Sensorelements 13 der Gruppenwiderstand der Widerstände RMinHc, RMaxHc sowie RHc des zweiten Gas-Sensorelements 13 stets innerhalb eines einfach zu berechnenden Bereiches bleibt. Hierdurch ist sichergestellt, daß sich die Frequenz des Ausgangssignals nicht in einen Bereich verschiebt, der sich außerhalb des Aufnahmebereichs der Auswertungseinheit 7 befindet.

Über den Widerstand RkHc kann die Grundfrequenz der zweiten Meßschaltung 20 in Abhängigkeit des zweiten Gas-Sensorelements 13 kalibriert werden.

Der Ausgang des dritten Operationsverstärkers 23 führt über die zweite Ausgangsleitung 14 zum Meßspannungsgenerator 2.

Der Meßspannungsgenerator 2 weist einen vierten Operationsverstärker 24 auf, dessen invertierender Eingang mit der zweiten Ausgangsleitung 14 in Verbindung steht. Im Rückführzweig zwischen seinem Ausgang und dem nicht-invertierenden Eingang ist ein Widerstand R5 sowie ein dazu parallel geschalteter Kondensator C2 vorgesehen. Der nicht-invertierende Eingang des vierten Operationsverstärkers 24 ist darüber hinaus über einen Widerstand R6 mit der Systemmasse verbunden.

Der vierte Operationsverstärker 24 bildet einen Fenster-Komparator mit Hysterese, der zwei Schaltschwellen aufweist. Der vierte Operationsverstärker 24 vergleicht somit die Spannungen an seinem invertierenden Eingang und an seinem nichtinvertierenden Eingang und schaltet entsprechend zum Ergebnis des Vergleichs seinen Ausgang auf einen jeweils unterschiedlichen, vorbestimmten Wert. Dabei bewirkt der Kondensator C2, daß sich der Ausgang des vierten Operationsverstärkers 24 schnell sättigt, was zu einem sauberen Grundsignal mit scharfen Flanken führt. Der Ausgang des vierten Operationsverstärkers 24 wird über die Grundsignalleitung 8 dem Meßspannungsumformungsblock 3 zugeführt.

Der Meßspannungsumformungsblock 3 weist eine Spule L1 auf, die in Reihe mit einem Widerstand R1 geschaltet ist. Dabei ist der Eingangsanschluß der Spule L1 mit der Grundsignalleitung 8 verbunden. Der Meßspannungsumformungsblock 3 weist weiterhin einen Widerstand R2 auf, der in Reihe mit dem Widerstand R1 geschaltet ist. Parallel zum Widerstand R2 ist ein Kondensator C1 geschaltet. Dabei ist der andere Anschluß des Widerstands R2 mit der Systemmasse verbunden. An dem Verbindungspunkt zwischen dem Widerstand R1 und dem Widerstand R2 ist zum einen eine Meßsignalleitung 9 vorgesehen, die mit dem nicht-invertierenden Anschluß des ersten Operationsverstärkers 21 verbunden ist. Weiterhin ist am Verbindungspunkt zwischen dem Widerstand R1 und dem Widerstand R2 eine Leitung vorgesehen, die über einen Widerstand R4 mit dem invertierenden Eingang des Operationsverstärkers 22 verbunden ist.

Zur Abtastung der Frequenz, mit der die zweite Meßschaltung 20 im Betrieb schwingt, ist die Auswertungseinheit 7 mit der Grundsignalleitung 8 verbunden.

Die zweite Meßschaltung 20 stellt einen für den Einsatz eines Doppelsensors geeigneten Oszillator unter anderem zur Auswertung der Güte von Luft dar.

Das Ausgangssignal des Meßspannungsgenerators 2 ist ein Rechtecksignal mit konstanter Amplitude, wobei dessen Amplitude der Amplitude der Versorgungsspannung entspricht, da es von einem Komparator erzeugt wird. Dieses Rechtecksignal wird mit Hilfe des Meßspannungsumformungsblocks 3 auf eine Amplitude von ca. 0,5 Volt abgeschwächt. Der Meßspannungsumformungsblock 3 stellt einen Tiefpassfilter zweiter Ordnung dar, der es erlaubt, auch die steilen Signalflanken des vom Meßspannungsgenerator 2 erzeugten Rechtecksignals abzuschwächen. Dieses Signal wird dem ersten Gas-Sensorelement 10 zugeführt. Am Gas-Sensorelement 10 liegt demzufolge eine definierte, konstante Wechselspannung an, die unabhängig von der umgebenden Gaszusammensetzung ist. Das Gas-Sensorelement 10 ist Bestandteil eines von dem ersten Operationsverstärker 21 gebildeten Integrators. Die Zeitkonstante dieses Integrators hängt in direkter Weise von dem Widerstand RNo des ersten Gas-Sensorelements 10 ab. Je kleiner der Widerstand RNo, um so schneller steigt die Spannungsrampe am Ausgang des ersten Operationsverstärkers 21 an. Die Rampe am Ausgang des ersten Operationsverstärkers 21 wird zu einem Komparator mit Hysterese geführt, der durch den vierten Operationsverstärker 24 gebildet wird. Da dessen Ausgang wiederum die Meßspannung der Gas-Sensorelemente 10 bzw. 13 steuert, ist die Schleife geschlossen und die Schaltung schwingt. Die Schwingfrequenz hängt in direkter Weise von der Schnelligkeit der Spannungsrampe am Verstärkerausgang des dritten Operationsverstärkers 23 und damit von dem Widerstand RHc des zweiten Gas-Sensorelements 13 ab, und darüber hinaus auch von der Schnelligkeit der Spannungsrampe am Ausgang des durch den ersten Operationsverstärker 21 gebildeten Integrators und damit vom Widerstand RNo des ersten Gas-Sensorelements 10. Die Ausgangsfrequenz ist nahezu proportional zum Verhältnis der Widerstände RNo/RHc.

Durch die Widerstände RMinNo, RMaxNo, RMinHc und RMaxHc wird sichergestellt, daß die zweite Meßschaltung 20 stets schwingt, auch wenn die Widerstände RNo und RHc wegen starker Luftbelastung aus dem schaltungstechnisch auswertbaren Widerstandsbereich geraten.

Das erste Gas-Sensorelement 10 kann dabei als $WO_3$-Sensor mit 20 μm Elektrodenabstand ausgebildet sein. Eine typische Betriebstemperatur des ersten Gas-Sensorelements 10 beträgt zwischen 300 und 350°C. Das zweite Gas-Sensorelement 13 kann als $SnO_2$-Sensor mit Aktivierungsschicht ausgebildet sein, dessen Betriebstemperatur zwischen 300 und 350°C beträgt. Alternativ dazu kann es auch als $Ga_2O_3$-Sensor mit einer Aktivierungsschicht ausgebildet sein, der bei einer Betriebstemperatur von 550 bis 600°C betrieben wird.

Die vom Meßspannungsumformungsblock 3 erzeugte Meßspannung kann trapezförmig oder sinusförmig sein oder anders geformt sein, z.B. mit runden Flanken.

Die vorstehend beschriebene zweite Meßschaltung 20 beschreibt einen für den Einsatz eines Doppelsensors geeigneten Oszillator unter anderem zur Auswertung der Güte von Luft. Dabei können zwei Gas-Sensorelemente verwendet werden, von denen das erste auf ein Gasgemisch mit $NO/NO_2$ reagiert und das sehr empfindlich auf Polarisationseffekte ist. Aus diesem Grund ist die Meßspannung an dem ersten Gas-Sensorelement 10 hinsichtlich der Amplitude und der Form der Meßspannung genau definierbar. So kann sie symmetrisch alternierend geformt sein und eine kleine, konstante Amplitude von ca. 0,5 Volt aufweisen.

Das zweite Gas-Sensorelement 13 reagiert auf ungesättigte Kohlenwasserstoffe und ist weniger empfindlich auf Polarisationseffekte, so daß die Amplitude der zur Messung verwendeten Wechselspannung nicht konstant sein muß, sondern beispielsweise zwischen 0,1 und 5 Volt schwanken kann. Die zweite Meßschaltung 20 liefert dann an der Grundsignalleitung 8 eine Frequenz, die proportional zum Verhältnis der Konzen-trationen von $NO/NO_2$ und Hc ist. Auf diese Weise verringert sich die Ausgangsfrequenz, wenn die $NO/NO_2$-Konzentration in der gemessenen Luft steigt, was bedeutet, daß der Widerstand des ersten Gas-Sensorelements 10 zunimmt. Dadurch können beispielsweise Abgase von Dieselfahrzeugen erkannt werden.

Die Ausgangsfrequenz auf der Grundsignalleitung 8 verringert sich ebenfalls, wenn die Konzentration von ungesättigten Kohlenwasserstoffen in einer gemessenen Luft steigt, weil dadurch der Widerstand RHc des zweiten Gas-Sensorelements 13 sinkt. Dies kann zur Erkennung der Abgase von Benzinfahrzeugen verwendet werden.

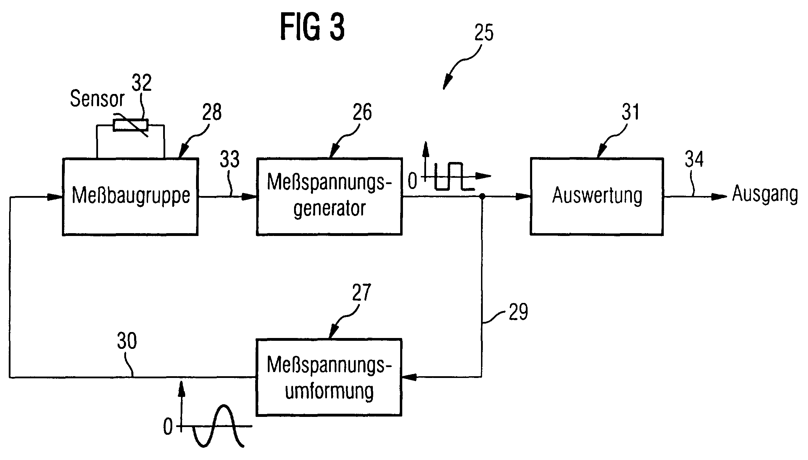

Figur 3 zeigt ein Blockschaltbild einer dritten Meßschaltung 25. Die dritte Meßschaltung 25 gliedert sich in einen Meßspannungsgenerator 26, in einen Meßspannungsumformungsblock 27 sowie in eine Meßbaugruppe 28. Dabei ist der Meßspannungsgenerator 26 über eine Grundsignalleitung 29 mit dem Meßspannungsumformungsblock 27 verbunden. Der Meßspannungsumformungsblock 27 steht über eine Meßsignalleitung 30 mit der Meßbaugruppe 28 in Verbindung. Von der Grundsignalleitung 29 wird eine Auswertungseinheit 31 mit einem Grundsignal auf der Grundsignalleitung 29 versorgt.

Die Meßbaugruppe 28 ist mit einem Gas-Sensorelement 32 versehen, mit dem die Konzentration eines Gases in einem Gasgemisch gemessen wird.

In der dritten Meßschaltung 25 erzeugt der Meßspannungsgenerator 26 ein Rechtecksignal auf der Grundsignalleitung 29, das als Grundsignal bezeichnet wird. Der Meßspannungsumformungsblock 27 formt dieses Grundsignal in ein Meßsignal um, dessen alternierende Wellenzüge Sinusform haben, und gibt dieses Sinussignal auf die Meßsignalleitung 30 ab, von wo es an die Meßbaugruppe 28 übermittelt wird. In der Meßbaugruppe 28 beaufschlagt das Meßsignal das Gas-Sensorelement 32, wodurch das Meßsignal verändert wird. Das veränderte Meßsignal wird über eine Ausgangsleitung 33 an den Meßspannungsgenerator 26 übermittelt.

Dadurch entsteht ein geschlossener Schwingkreis, der mit einer Frequenz schwingt, die von dem Widerstand des Gas-Sensorelements 32 abhängt. Mit der Auswertungseinheit 31 wird die jeweilige Frequenz bestimmt, mit der die dritte Meßschaltung 25 schwingt. Das Meßergebnis wird an einer Auswertungsleitung 34 bereitgestellt, wo es zur Steuerung beispielsweise einer Klimaanlage abgegriffen werden kann.

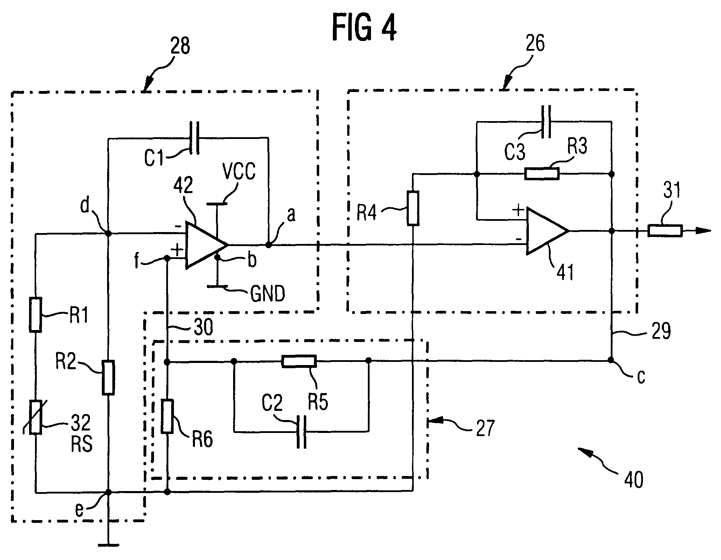

Figur 4 zeigt ein Schaltbild einer vierten Meßschaltung 40, die im wesentlichen der Meßschaltung aus Figur 3 entspricht. Gleichen Baugruppen und gleichen Bauteilen sind daher dieselben Bezugsziffern gegeben.

Der Meßspannungsgenerator der vierten Meßschaltung 40 weist einen ersten Operationsverstärker 41 auf, in dessen Rückführzweig zwischen Ausgang und nicht-invertierendem Eingang ein Widerstand R4 sowie ein Kondensator C3 geschaltet sind. Der erste Operationsverstärker 41 stellt einen Komparator mit Hysterese dar. Der Ausgang des ersten Operationsverstärkers 41 ist mit der Grundsignalleitung 29 verbunden, an die wiederum die Auswertungseinheit 31 angeschlossen ist. Der nichtinvertierende Eingang des ersten Operationsverstärkers 41 ist über einen Widerstand R4 mit einer virtuellen Systemmasse "e" verbunden. Die Grundsignalleitung 29 wird in den Meßspannungsumformungsblock 27 eingespeist, der einen Widerstand R5 mit dazu parallel geschaltetem Kondensator C2 aufweist. Der Widerstand R5 ist über einen Widerstand R6 mit der virtuellen Systemmasse "e" verbunden. Somit ist der Meßspannungsumformungsblock 27 als Filter ausgebildet, der ein Meßsignal auf der Meßsignalleitung 30 abgibt, die zu der Meßbaugruppe 28 führt.

Die Meßbaugruppe 28 beinhaltet einen als Integrator geschalteten zweiten Operationsverstärker 42, dessen Versorgungsspannungsanschlüsse zwischen einer absoluten Masse GND und einer Versorgungsspannung Vcc geschaltet sind. Der nichtinvertierende Eingang des zweiten Operationsverstärkers 42 ist mit der Meßsignalleitung 30 verbunden.

In einem Rückführzweig zwischen dem Ausgang und dem invertierenden Eingang des zweiten Operationsverstärkers 42 ist eine Kapazität C1 geschaltet. Weiterhin erstreckt sich zwischen dem invertierenden Eingang des zweiten Operationsverstärkers 42 und der virtuellen Systemmasse "e" ein Widerstand R2, zu dem parallel eine Reihenschaltung aus dem Gas-Sensorelement 32 und einem Widerstand R1 geschaltet ist.

Im Betrieb verhält sich die dritte Meßschaltung 40 wie folgt. Am invertierenden Eingang des zweiten Operationsverstärkers 42 wird die Meßspannung konstant gehalten, wobei der nichtinvertierende Eingang des zweiten Operationsverstärkers 42 auf diesen Wert nachgeregelt wird. Dabei darf durch den nicht-invertierenden Eingang des zweiten Operationsverstärkers 42 kein Polarisationsstrom fließen, da ansonsten dieser Strom einerseits das Ausgangssignal des zweiten Operationsverstärkers 42 verfälschen kann und andererseits das Gas-Sensorelement 32 polarisieren kann. Vorzugsweise wird ein J-FET-Operationsverstärker benutzt, bei dem sich der Polarisationsstrom im pA-Bereich befindet. Dieser Strom beträgt ca. 20 pA - 200 pA und er ist temperaturabhängig in der Größenordnung von ca. 0,2% des Meßstroms. Im Ausgangspunkt beträgt die Ausgangsspannung der Schaltung Vcc und alle Kondensatoren sind entladen.

Am nicht-invertierenden Eingang des zweiten Operationsverstärkers 42 erscheint eine Spannung $U_{fb} = Vcc/2 * (1 + R5/R6)$. Der zweite Operationsverstärker 42 steuert seinen Ausgang so, daß die Spannungsdifferenz zwischen den zwei Eingängen bei 0 V bleibt. Wenn ein konstanter Widerstand RS an die Schaltung angeschlossen ist, hat die Ausgangsspannung des zweiten Operationsverstärkers 42 die folgende Form:

$$U_{ab} = \frac{Vcc}{2} * U_f e * \left(1 + \frac{t}{R2//(R1+RS)}\right)$$

Diese Spannung steigt dann linear an. Der erste Operationsverstärker 41 ist als Komparator mit Hysterese geschaltet. Die Schwelle, bei der sein Ausgang nach 0 V kippt, beträgt

$$V_{Komparator +} = \frac{Vcc}{2} * \left(1 + \frac{R4}{R3+R4}\right)$$

Nachdem die Spannung $U_{ab}$ diese Schwelle überschritten hat, kippt der Ausgang des ersten Operationsverstärkers 41 nach 0 V. Die Spannung am nicht-invertierenden Eingang des zweiten Operationsverstärkers 42 beträgt dann

$$U_{fee} = \frac{Vcc}{2} * \left(1 - \frac{R5}{R6}\right)$$

Der Kondensator C1 entlädt sich langsam wieder und die Spannung $U_{ab}$ fällt linear mit der Zeit ab. Ist die untere Schwelle des ersten Operationsverstärkers 41

$$V_{Komparator-} = \frac{Vcc}{2} * \left(1 + \frac{R4}{R3+R4}\right)$$

unterschritten, so kippt der Ausgang des ersten Operationsverstärkers 41 wieder nach Vcc. Die Schaltung befindet sich wieder im Anfangszustand und beginnt zu schwingen. Die Schwingungsfrequenz hängt dabei von dem Wert RS des Gas-Sensorelements 32 ab.

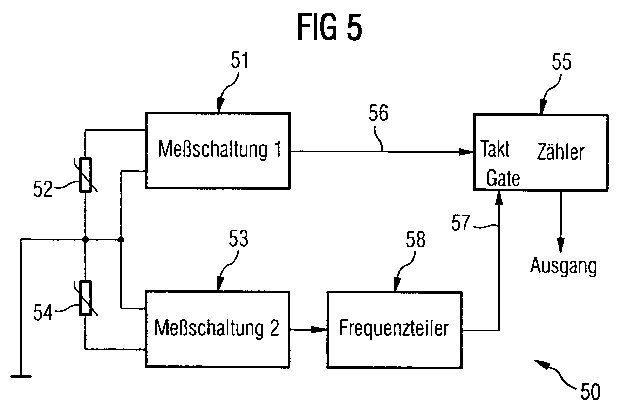

Figur 5 zeigt eine vierte Meßschaltung 50 in der Ansicht als Blockschaltbild.

Die vierte Meßschaltung 50 weist eine erste Untermeßschaltung 51 mit einem ersten Gas-Sensorelement 52 sowie eine zweite Untermeßschaltung 53 mit einem zweiten Gas-Sensorelement 54 auf. Die erste Untermeßschaltung 51 und die zweite Untermeßschaltung 53 entsprechen im wesentlichen der vierten Meßschaltung 40 aus Figur 4.

Weiterhin ist ein Zählerbaustein 55 vorgesehen, der einen Takteingang 56 sowie einen Gate-Eingang 57 aufweist. Der Ausgang der ersten Untermeßschaltung 51 ist direkt in den Takteingang 56 eingespeist. Der Ausgang der zweiten Untermeßschaltung 53 ist einem Frequenzteiler 58 zugeführt, dessen Ausgang dem Gateeingang 57 zugeführt ist.

Im vorliegenden Fall ist der Zähler 55 als Zählerteil eines Mikrocontrollers ausgebildet. Das erste Gas-Sensorelement 52 und das zweite Gas-Sensorelement 54 können entweder als identische Sensoren eingesetzt werden, wobei ein Sensor passiviert ist, so daß Umgebungsgase nicht zu seiner Sensorschicht gelangen können. Mit einem solchen Aufbau kann mit der fünften Meßschaltung 50 eine relativ temperaturunempfindliche Messung von Gaskonzentrationen durchgeführt werden. Da beide Sensoren vorzugsweise dieselbe Aktivierungsenergie aufweisen, folgt nämlich auch das Temperaturverhalten der Gas-Sensorelemente derselben Funktion.

Der Frequenzteiler 58 teilt das Ausgangssignal der zweiten Untermeßschaltung 53 mit einem Teilungsfaktor n. Somit entspricht der Zählerstand des Zählers 55 direkt dem Verhältnis der Frequenzen der ersten Untermeßschaltung 51 und der zweiten Untermeßschaltung 53 multipliziert mit n. Unter der Voraussetzung, daß die erste Untermeßschaltung 51 und die zweite Untermeßschaltung 53 eine lineare Abhängigkeit zwischen der jeweiligen Leitfähigkeit der Gas-Sensorelemente 52 bzw. 54 und der Ausgangsfrequenz aufweisen, ist das digitale Ergebnis proportional zum Verhältnis der Widerstände des ersten Gas-Sensorelements 52 und des zweiten Gas-Sensorelements 54. Dieses Zählerergebnis kann direkt als temperaturunempfindliches und lediglich gasempfindliches Signal behandelt werden.

Wenn das erste Gas-Sensorelement 52 und das zweite Gas-Sensorelement 54 als Gas-Sensorelemente vorgesehen werden, die auf unterschiedliche Gase eines Gasgemisches reagieren, dann kann auf die gleiche Weise eine Kompensation eines Maskierungseffektes bewirkt werden.

Bei der fünften Meßschaltung 50 aus Figur 5 ist es notwendig, die erste Untermeßschaltung 51 und die zweite Untermeßschaltung 53 durch Abschirmung voneinander zu trennen, so daß keine Synchronisation zwischen der ersten Untermeßschaltung 51 und der zweiten Untermeßschaltung 53 erfolgen kann.

**Patentansprüche**

1. Meßschaltung zur Ermittlung der Konzentration eines oder mehrerer Gase in einem Gasgemisch, die die folgenden Merkmale aufweist:

   - einen Schwingkreis mit veränderlicher Eigenfrequenz, der wenigstens ein Gas-Sensorelement (10, 13; 32; 52, 54) mit variablem Widerstand umfaßt, wobei sich der Widerstand des Gas-Sensorelements (10, 13; 32; 52, 54) in Abhängigkeit der Konzentration des Gases bzw. der Gase in dem Gasgemisch verändert,
   - wenigstens einen Signalgenerator (2; 26), der das Gas-Sensorelement (32) bzw. die Gas-Sensorelemente (10, 13; 52, 54) mit einem alternierenden Meßsignal beaufschlagt, dessen Grundfrequenz von der Größe des Widerstands des Gas-Sensorelements (32) bzw. der Gas-Sensorelemente (10, 13; 52, 54) abhängt,

   wobei der Signalgenerator (2; 26) so ausgebildet ist, daß das Gas-Sensorelement (32) bzw. die Gas-Sensorelemente (10, 13; 52, 54) mit einem Meßsignal beaufschlagbar ist, dessen Ableitung nach der Zeit im wesentlichen stetig verläuft.

2. Meßschaltung nach Anspruch 1,
   **dadurch gekennzeichnet, daß**
   der Signalgenerator (2; 26) so ausgebildet ist, daß das Gas-Sensorelement (32) bzw. die Gas-Sensorelemente (10, 13; 52, 54) mit einem Meßsignal beaufschlagbar ist, dessen Ableitung nach der Zeit über den Meßbereich des Gas-Sensorelements (32) bzw. des Gas-Sensorelemente (10, 13; 52, 54) im wesentlichen stetig verläuft.

3. Meßschaltung zur Ermittlung der Konzentration eines oder mehrerer Gase in einem Gasgemisch, die die folgenden Merkmale aufweist:

   - einen Schwingkreis mit veränderlicher Eigenfrequenz, der wenigstens ein Gas-Sensorelement (10, 13; 32; 52, 54) mit variablem Widerstand umfaßt, wobei sich der Widerstand des Gas-Sensorelements (32) bzw. der Gas-Sensorelemente (10, 13; 52, 54) in Abhängigkeit der Konzentration des Gases bzw. der Gase in dem Gasgemisch verändert,
   - wenigstens einen Signalgenerator (2; 26), der das Gas-Sensorelement (32) bzw. die Gas-Sensorelemente (10, 13; 52, 54) mit einem alternierenden Meßsignal beaufschlagt, dessen Grundfrequenz von der Größe des Widerstands des Gas-Sensorelements (32) bzw. der Gas-Sensorelemente (10, 13; 52, 54) abhängt,

**dadurch gekennzeichnet, daß**
der Signalgenerator (2; 26) so ausgebildet ist, daß das Gas-Sensorelement (32) bzw. die Gas-Sensorelemente (10, 13; 52, 54) mit einem Meßsignal beaufschlagbar ist, dessen Amplitude und/oder Form über den Meßbereich des Gas-Sensorelements (32) bzw. der Gas-Sensorelemente (10, 13; 52, 54) im wesentlichen konstant ist.

4. Meßschaltung zur Ermittlung der Konzentration eines oder mehrerer Gase in einem Gasgemisch, die wenigstens ein Gas-Sensorelement (10, 13; 32; 52, 54) mit variablem Widerstand aufweist, dessen Widerstand sich in Abhängigkeit der Konzentration eines Gases in dem Gasgemisch verändert, wobei die Meßschaltung die folgenden Stufen aufweist:

   - eine Meßsignalerzeugungsstufe (2; 26) zur Erzeugung eines alternierenden Grundsignals,
   - eine Meßsignalumwandlungsstufe (3; 27) zur Umwandlung des Grundsignals in ein Meßsignal, und
   - eine Meßstufe (4, 6; 28) mit dem Gas-Sensorelement (10, 13; 32; 52, 54),

   wobei ferner die Meßsignalerzeugungsstufe (2; 26) und/oder die Meßsignalumwandlungsstufe (3; 27) und die Meßstufe (4, 6; 28) so zusammengeschaltet sind, daß diese einen Schwingkreis bilden, dessen charakteristische Schwingungseigenschaften sich mit der Veränderung des Widerstands des Gas-Sensorelements (10, 13; 32; 52, 54) ändert.

5. Meßschaltung nach Anspruch 4,
   **dadurch gekennzeichnet, daß**
   die Meßsignalerzeugungsstufe (2; 26) einen als Fensterkomparator mit Hysterese ausgebildeten Operationsverstärker (24; 41) aufweist.

6. Meßschaltung nach Anspruch 4 oder Anspruch 5,
   **dadurch gekennzeichnet, daß**
   die Meßsignalumwandlungsstufe (3; 27) als Bandpaßfilterstufe (L1, C1, R1, R2) ausgebildet ist.

7. Meßschaltung nach einem der Ansprüche 4 bis 6,
   **dadurch gekennzeichnet, daß**
   die Meßstufe (4; 28) einen als Integrator geschalteten Operationsverstärker (21; 42) aufweist, dessen erster Eingang mit dem Gas-Sensorelement (10; 32; 52, 54) verbunden ist und dessen zweiter Eingang mit dem Ausgang der Meßsignalumwandlungsstufe (3; 27) verbunden ist.

8. Meßschaltung nach Anspruch 9,
   **dadurch gekennzeichnet, daß**
   das Gas-Sensorelement als hochohmiges Halbleitergassensorelement (10; 32; 52, 54) ausgebildet ist.

9. Meßschaltung nach Anspruch 7 oder Anspruch 8,
   **dadurch gekennzeichnet, daß**
   in einem Rückführzweig zwischen dem Ausgang
   und einem Eingang des Operationsverstärkers (21;
   42) eine Kapazität (CkNo; C1) vorgesehen ist.

10. Meßschaltung nach einem der Ansprüche 4 bis 6,
    **dadurch gekennzeichnet, daß**
    die Meßstufe (6) einen als Verstärker geschalteten
    Operationsverstärker (23) aufweist, dessen erster
    Eingang mit dem Ausgang der Meßsignalumwandlungsstufe (3; 27) verbunden ist, wobei in einem
    Rückführzweig zwischen dem Ausgang und einem
    Eingang des Operationsverstärkers (23) ein Gas-
    Sensorelement (13) vorgesehen ist.

11. Meßschaltung nach Anspruch 10,
    **dadurch gekennzeichnet, daß**
    das Gas-Sensorelement als niederohmiges Halbleitergassensorelement (13) ausgebildet ist.

12. Meßschaltung zur Ermittlung der Konzentration
    eines oder mehrerer Gase in einem Gasgemisch,
    die ein erstes Gas-Sensorelement (10) mit variablem Widerstand sowie ein zweites Gas-Sensorelement (13) mit variablem Widerstand aufweist,
    deren Widerstände sich jeweils in Abhängigkeit der
    Konzentration unterschiedlicher Gase in dem Gasgemisch verändern,
    wobei die Meßschaltung die folgenden Stufen aufweist:

    - eine Meßsignalerzeugungsstufe (2) zur Erzeugung eines alternierenden Grundsignals, und
    - eine Meßsignalumwandlungsstufe (3) zur
      Umwandlung des Grundsignals in ein Meßssignal,
    - eine erste Meßstufe (4) mit dem ersten Gas-
      Sensorelement (10),
    - eine zweite Meßstufe (6) mit dem zweiten Gas-
      Sensorelement (13),

    wobei weiterhin die Meßsignalerzeugungsstufe (2)
    und/oder die Meßsignalumwandlungsstufe (3)
    sowie die erste Meßstufe (4) und die zweite Meßstufe (6) so zusammengeschaltet sind, daß diese
    einen Schwingkreis bilden, dessen charakteristische Schwingungseigenschaften sich mit der Veränderung des Widerstands des ersten Gas-
    Sensorelements (10) und/oder des zweiten Gas-
    Sensorelements (13) ändert.

13. Meßschaltung nach Anspruch 12,
    **dadurch gekennzeichnet, daß**
    wobei ferner der Ausgang der Meßsignalumwandlungsstufe (3) mit dem Eingang der ersten Meßstufe (4) in Verbindung steht und der Ausgang der
ersten Meßstufe (4) mit dem Eingang der zweiten
Meßstufe (6) in Verbindung steht.

14. Meßschaltung nach Anspruch 13,
    **dadurch gekennzeichnet, daß**
    zwischen erster Meßstufe (4) und zweiter Meßstufe
    (6) eine Potentialanpassungsstufe (5) vorgesehen
    ist, die so ausgebildet ist, daß ein Anteil des Meßsignals am Ausgang der ersten Meßstufe (4) unterdrückbar ist.

15. Meßschaltung nach Anspruch 14,
    **dadurch gekennzeichnet, daß**
    die Potentialanpassungsstufe (5) einen als Subtrahierer geschalteten zweiten Operationsverstärker
    (22) aufweist, dessen erster Eingang mit dem Ausgang der ersten Meßstufe (4) verbunden ist und
    dessen zweiter Eingang mit dem Ausgang der
    Meßsignalumwandlungsstufe (3) verbunden ist,
    wobei in einem Rückführzweig zwischen dem Ausgang und einem Eingang des zweiten Operationsverstärkers (22) ein Widerstand (R3) vorgesehen
    ist.

## FIG 1

Sensor 1

Meßbaugruppe Sensor 1 — 4

Potential-anpassung — 5

Sensor 2

Meßbaugruppe Sensor 2 — 6

Meßspannungs-generator — 2

Auswertungs-einheit (f) — 7

Ausgang

Meßspannungs-umformung — 3

10

11

12

13

14

8

15

9

0

1

EP 0 942 281 A2

FIG 2

Meßsignal
(Freqenz)

# FIG 3

EP 0 942 281 A2

# FIG 4

# FIG 5